# EUROPEAN PATENT APPLICATION

(11) **EP 1 475 054 A2**
(43) Date of publication of application: **10.11.2004**
(21) Application number: 04252580.8
(22) Date of filing: 04.05.2004
(51) Int. Cl.: A61F 2/06

(54) **Means and method for stenting bifurcated vessels**

(30) Priority: 05.05.2003 US 467934 P
(71) Applicant: Cordis Corporation, Miami Lakes Florida 33014 (US)
(72) Inventor: Fischell, Robert E., Dayton, MD 21036 (US); Burgermeister, Robert, Bidgewater, NJ 08807 (US); Fischell, David R., Fair Haven, NJ 07704 (US); Fischell, Tim A., Richland, MI 49083 (US); Trotta, Thomas N., Aventura, FL 33180 (US); Sidwall, Scott, Hollywood, FL 33019 (US)
(74) Representative: Belcher, Simon James

(57) **Abstract**

A system for placing a stent into the side branch of an arterial bifurcation. The system includes:
(i) An inflatable balloon (15') located at a distal portion of a stent delivery system (16'), the stent delivery system having an outer shaft (11) with a longitudinal length, the outer shaft having a distal end that is fixedly attached to the proximal end of the balloon.
(ii) A pre-deployed side branch stent (14) mounted coaxially onto the balloon for placement into the side branch of an arterial bifurcation, the stent having a proximal end and a distal end.
(iii) A guide wire (6); and
(iv) A main guide wire tube (12) having a proximal end and a distal end and having an interior lumen (19) that allows the stent delivery system to be moved slideably over the guide wire, the main guide wire tube being fixedly attached to the balloon at a location that is in close proximity to the proximal end of the stent.

## Description

This invention is in the field of devices to restore normal blood flow in a bifurcated vessel that has a stenosis at its bifurcation.

It has been shown that intravascular stents are an excellent means to maintain the patency of blood vessels following balloon angioplasty. As stent technology has advanced, increasingly complex anatomy has been treatable with stents. A particularly difficult anatomy to treat is that of a bifurcation in a blood vessel or the ostium of a side branch. In US-5749825 by Fischell et al, a stent and stent delivery system are described for stenting bifurcations. The Fischell design has two guide wire lumens allowing the deployment of a stent in the main blood vessel while leaving a guide wire positioned through the stent struts into a side branch vessel. First, by needing two guide wires and a stent for the Fischell design, the profile (outside diameter) of the stenting system is significantly larger than a single guide wire stent. Currently available stents like the stent sold under the mark Cordis BX Velocity (Miami Lakes, Florida) stent that utilize only a single guide wire, have sufficiently large cells that a guide wire can almost always be placed through the stent into the side branch after the stent has been deployed. Second, the Fischell design addresses the issue of placing a stent into the proximal artery and main branch. It does not concern itself with the placement of a stent into the side branch. Still further the Fischell design does not address the frequent problem of restenosis at the ostium, which frequently occurs when stenting at a bifurcation using a stent that is not drug eluting.

A "Y"-shaped bifurcation stent with two distal balloons and stent segments for stenting each of the vessels would address the issue of stenting the second branch vessel but such a device would be even larger in profile and harder to deliver than the Fischell device described in US-5749825. If one places a standard stent across the ostium of the side branch and the side branch is not at a 90-degree angle to the main branch, then either the second stent will extend into the main branch or some portion of the ostium will not be properly supported by the second stent.

Most current tubular stents use a multiplicity of circumferential sets of strut members connected by either straight longitudinal connecting links or undulating longitudinal flexible links. The circumferential sets of strut members are typically formed from a series of straight diagonal sections connected to curved sections forming a circumferential, closed-ring, zig-zag structure. This structure opens up as the stent expands to form the element of the stent that provides structural support for the arterial wall. A "single strut member" is defined herein as a diagonal section connected to a curved section within one of the circumferential sets of strut members.

Fischell et al., in US-2003/0050688-A1 describes a stent with an angled proximal circumferential set of strut members. While this stent is ideally suited to scaffold the ostium of a side branch, delivery by a standard balloon could be difficult as the unwrapping of a folded balloon can rotate the stent during deployment, making alignment of the most proximal circumferential set of strut members with the ostium difficult.

For the purposes of this disclosure, the term "side branch" will refer to either a branch vessel that connects into the side of a larger vessel or the smaller of the two vessels that join to an arterial bifurcation. An "arterial bifurcation" will refer to any split of the blood flow from one lumen into two lumens.

The present invention consists of a main artery stent (the main stent) that is placed in the main artery and a second stent that is placed into the side branch (the side branch stent), the two stents constituting a complete treatment for a stenosed arterial bifurcation. The stents are preferably drug eluting. The main stent would optimally be one that has a reasonably small area of each cell after the stent is deployed, but also has a large perimeter length for each cell. The small cell area allows for optimizing the uniform elution of a drug such as sirolimus, which has been shown to be an effective means for reducing restenosis after implantation of such a drug eluting stent. The long length of the perimeter of each cell is important for forming a comparatively large, near-circular opening at the ostium of the side branch after the main stent is deployed. This opening would be formed after placing a guide wire and then a balloon through the side of the main stent at the site of the ostium of the side branch and then inflating the balloon. Dilating the struts at the side of the main stent "unjails" the side branch and prepares the main stent for the introduction of the side branch stent into the side branch artery. Optimally, the unjailing of the side branch would be achieved without breakage of any strut of the main stent. Fischell et al in US-6540775, which is similarly included herein by reference, describe an ideal design for a main stent that can be inserted at an arterial bifurcation.

An optimum stent design for stenting the side branch of the bifurcation is generally described by Fischell et al in US-2003/0050688-A1.

The role of the side branch stent is to form a scaffold to open the ostium and near-ostium region of the side branch of the bifurcation without extending into the main artery and also providing complete coverage of the wall of the side branch in the region of its ostium. As described by Fischell in US-2003/0050688-A1, the angulated side branch stent would have multiple sets of circumferential strut members with the most distal set of strut members being similar to that of most stents in that the plane of the distal end circumferential set of strut members is perpendicular to the stent's longitudinal axis. Only the most distal set of strut members should have its plane perpendicular to the stent's longitudinal axis although typically there would be several such circumferential sets of strut members at the distal potion of the stent. As one move longitudinally in a proximal direction from the distal portion of the side branch stent, the circumferential sets of strut members would become increasingly angulated. The most proximal set of strut members would have the greatest angulation relative to the stent's longitudinal axis. The plane of the most proximal circumferential set of strut members would have an angle between 30-75 degrees relative to the stent's (and the balloon's) longitudinal axis when the stent is expanded. The angle that would be used would depend upon the angle of the centerline of the side branch artery relative to a centerline of the main artery. For coronary arteries the angle between the main branch and the side branch is preferably 60±10 degrees. It is also envisioned to widen the diagonal struts for the most proximal and most distal circumferential sets of strut members to increase their radiopacity and to allow for more drug to be placed at the ends of the stent to reduce any tendency for edge restenosis after stent implantation. It is also anticipated that the region of the side branch stent that is placed near the ostium of the side branch could have an increased level of drug concentration as the means to provide a higher total dose of drug to prevent restenosis in that most sensitive region of the bifurcation where restenosis often occurs.

A very important aspect of the design of the present invention is the stent delivery system for the side branch stent that tracks over two guide wires (one in the main branch and one in the side branch). The use of two guide wires guarantees the longitudinal position and prevents rotation of the stent's angled proximal end during balloon inflation.

The balloon of the present invention stent delivery system may have a primary guide wire lumen (for the side branch guide wire) extending throughout the length of the balloon. The second guide wire lumen (for the main branch guide wire) can be provided by a short, flexible guide wire tube that is attached onto or into the balloon from a point just proximal to the proximal end of the stent and extending in the proximal direction for some length along the balloon. This tube could also extend for a distance in a proximal direction along the outer shaft of the stent delivery system for the side branch stent. The guide wire tube, having a distal opening that is outside of the balloon, would fit slideably around the main guide wire, the main guide wire being placed into and through the main artery branch. An important purpose of the side branch guide wire and the main guide wire is to orient the side branch stent as it is advanced into the bifurcation so as to force a proper angular orientation of the side branch stent at the ostium of the side branch artery. Another purpose of the main guide wire and the guide wire tube attached to the balloon is to assure the correct longitudinal positioning of the side branch stent relative to the ostium of the side branch artery. That is, it is optimal to prevent the proximal end of the side branch stent from extending into the main stent and also optimum to not leave any significant area of the ostium of the side branch uncovered by stent struts. It is particularly important to have a reasonably high level of drug elution at the ostium of the side branch to address a tendency of restenosis at the bifurcation region.

Another important aspect of the design of the stent delivery system for the side branch stent is the shape and method of folding the balloon onto which the stent is mounted. The side branch stent delivery system and the main guide wire are designed to cooperate to obtain the proper orientation and longitudinal position of the side branch stent prior to side branch stent deployment. However, if the balloon onto which the side branch stent is mounted can twist as the stent is deployed, it could misalign the orientation of the most proximal circumferential set of strut members of the side branch stent with respect to the ostium of the side branch. It should be noted that a certain amount of twisting of the stents with respect to the balloon is acceptable. Yet, it may be important that the balloon of the stent delivery system for the side branch stent not cause any significant additional rotation of the side branch stent after its correct initial angular orientation has been achieved by the two guide wire stent delivery system. To prevent rotation of the side branch stent as it deploys outwardly into the side branch artery, it may be required that the balloon on which the stent is mounted be folded in a symmetric manner. Specifically, there must be as many folds of the folded balloon that fold in a clockwise direction as compared to the number of folds that fold in a counter-clockwise direction. For example, to prevent stent rotation during balloon inflation, there may be a total of four balloon folds; two folded clockwise and two-folded counter clockwise.

An alternate means for preventing stent rotation as the balloon is inflated would be to surround the folded balloon with a thin-walled elastomer tube that would expand in an elastic manner as the balloon is inflated without rotating about the balloon's longitudinal axis. With sufficient lubricity between the outer surface of the folded balloon and the inner surface of the elastomer tube, the balloon could be expanded with the elastomer tube not rotating and therefore preventing the stent mounted on the elastomer tube from rotating. It is also envisioned that a strong elastic balloon material like Gore-Tex ® described in, among other references, US-5752934, might be used where no folds are required.

Although one method for stenting a bifurcation stenosis is to first place the main stent and then the side branch stent, it should be understood that a desirable alternative method is to first place the side branch stent and then the main stent. If the side branch stent is placed first, then the main stent can provided structural support for that part of the side branch stent that covers the obtuse point of the side branch.

Another preferred embodiment of this invention is to have first and second portions of the main guide wire tube with the distal end of the first portion attached continuously to the proximal end of the second portion. The first portion would be attached to the balloon of the stent delivery system (and possibly also attached to the outer shaft) with the distal end of the attached portion being situated just proximal to the proximal end of the stent. The second portion of the main guide wire tube would extend continuously from that distal end of the attached portion for a distance of approximately 2 mm to 30 mm. This second portion would not be attached to the balloon or any other part of the stent delivery system. The second portion would provide a better means for accurately locating the side branch stent at the arterial side branch by reinforcing the strength of the main guide wire. A longer guide wire tube would also provide better tracking over the main guide wire.

This invention provides a means and method for stenting of a bifurcated artery of a human subject. The means including both a main stent and a side branch stent that is delivered after the main stent has been deployed in the main artery of a bifurcated artery. The side branch stent is delivered into the side branch artery through a dilated opening in the side of the main stent.

Also, this invention provides a main stent that has a comparatively small area of each cell after deployment of the stent but a comparatively long perimeter length for each cell.

Still further, this invention provides a side branch stent that has circumferential sets of strut members that become increasingly angulated relative to the longitudinal axis of the balloon on which the stent is mounted as one moves from the stent's distal end toward the stent's proximal end.

Also, this invention provides a stent delivery system for the side branch stent that includes a flexible guide wire tube that is designed to have the main guide wire pass through it into the main artery branch of the bifurcation.

Again, this invention uses the cooperation of the side branch guide wire and the main guide wire as it passes through the main guide wire tube to properly orient the side branch stent in the side branch artery of the bifurcation as the side branch stent delivery system is advanced through the arterial bifurcation, proper orientation being the placement of the plane of the most proximal circumferential set of strut members of the side branch stent being placed essentially co-planar with the plane of the ostium of the side branch artery.

Further, the stent delivery system of the side branch stent is designed to cooperate with the main guide wire for accurate longitudinal placement of the side branch stent so that the most proximal circumferential set of strut members is placed generally at the ostium of the side branch artery.

Yet again, this invention provides a main guide wire tube that is fixedly attached to the balloon of the side branch stent being highly radiopaque to assist in obtaining the proper angular orientation of the side branch stent into the side branch of the bifurcation.

Both the main stent and the side branch stent each include a means for eluting a drug into the arterial walls at the site of the bifurcation, thereby minimizing any tendency for restenosis at the bifurcation.

Both the main stent and the side branch stent each having end circumferential sets of strut members that have an increased strut width, so as to provide increased drug elution at the ends of the stents thereby reducing any tendency for restenosis beyond the edges of each stent.

The balloon of the stent delivery system for the side branch stent is designed to overcome angular rotation of the side branch stent as the stent is deployed into the side branch artery.

A method for stenting a side branch is shown comprising: first stenting the main artery, then placing a guide wire through the main stent's struts into the side branch, dilating with a balloon catheter to unjail the ostium of the side branch, inserting a side branch stent mounted on a balloon into the side branch, and deploying the side branch stent into the side branch artery.

Alternately, a method is disclosed by which the user first places the side branch stent and then places the main stent.

Furthermore, a main guide wire tube has a first portion that attaches to the balloon and a second more distal portion that is not attached to the balloon, the second portion being between approximately 2 and 30 mm in length.

The system of the invention can be used in a method for stenting an arterial bifurcation, in a human subject, the method including the following steps:
a. placing a main guide wire into and through a proximal artery and a main branch of that artery;
b. advancing a stent delivery system that includes a main branch stent over the guide wire and deploying the main stent with its longitudinal center located approximately over the ostium of a side branch artery of the proximal artery and removing the stent delivery system from the subject;
c. inserting a side branch guide wire through the side of the deployed main stent;
d. advancing a balloon angioplasty catheter over the side branch guide wire until a balloon located at a distal portion of the balloon angioplasty catheter is approximately centered within the ostium of the side branch artery and inflating the balloon to open the struts of the main stent to form an opening at the ostium of the side branch artery that is approximately the same area as the ostium of the side branch artery;
e. deflating the balloon from step d) above and removing the balloon angioplasty catheter from the human subject;
f. advancing a stent delivery system having two guide wire lumens over both the side branch guide wire and the main guide wire, the stent delivery system having an inflatable balloon and a side branch stent both located at a distal portion of the stent delivery system, the stent delivery system also having a main guide wire tube whose lumen is one of the two guide wire lumens of the stent delivery system, at least part of the main guide wire tube being fixedly attached to the balloon at a location that is in close proximity to the proximal end of the side branch stent, the side branch guide wire passing through the inner shaft of the stent delivery system and the main guide wire passing through the main guide wire tube that is fixedly attached to the balloon;
g. advancing the stent delivery system for the side branch stent until the stent's proximal end is placed in close proximity to the carina of the bifurcation;
h. inflating the balloon so as to deploy the side branch stent against the walls of the side branch artery of the bifurcation;
i. deflating the balloon and removing the side branch stent delivery system from the human subject; and
j. removing the side branch guide wire and the main guide wire from the human subject.

Preferably, the side branch stent is an angulated side branch stent.

The system of the invention can also be used in method for stenting an arterial bifurcation of a human subject, the method including the following steps:
a. inserting a side branch guide wire into and through the ostium of a side branch of a bifurcated artery;
b. placing a main guide wire into and through a proximal artery and main branch artery of the bifurcation;
c. advancing a side branch stent delivery system that includes a side branch stent having an attached main guide wire tube over the side branch guide wire with the main guide wire passing through the main guide wire tube, the side branch stent being advanced until its proximal end is in close proximity to the carina of the side branch;
d. deploying the side branch stent into the side branch artery and removing the side branch stent delivery system and the side branch guide wire from the human subject;
e. advancing a main stent delivery system that includes a main stent over the main guide wire and deploying the main stent with its longitudinal center located approximately over the ostium of the side branch artery and removing the main stent delivery system from the human subject;
f. advancing a side branch guide wire through the side of the deployed main stent at the site of the ostium of the side branch artery;
g. advancing a balloon angioplasty catheter over the side branch guide wire until a balloon located at a distal portion of the balloon angioplasty catheter is approximately centered within the ostium of the side branch artery and inflating the balloon to open the struts of the main stent to form an opening at the ostium of the side branch artery that is approximately the same area as the ostium of the side branch artery; and
h. deflating the balloon from step g) and removing both the balloon angioplasty catheter and the side branch guide wire from the human subject.

Preferably, the side branch stent is an angulated side branch stent.

Preferably, the main guide wire tube has a proximal portion that is attached to the balloon of the side branch stent delivery system and main guide wire tube has a distal portion that extends freely from the distal end of the proximal portion of the main guide wire tube and is not directly attached to any part of the side branch stent delivery system.

These and other objects and advantages of this invention will become apparent to a person of ordinary skill in this art upon reading the detailed description of this invention including the associated drawings as presented herein.

Embodiments of the invention will now be described by way of example with reference to the accompanying drawings, in which:
FIG. 1 is a cross section of a bifurcated artery showing plaque deposits on the arterial walls and a main guide wire placed through the proximal artery into the main branch.
FIG. 2 is a cross section of the main stent placed inside the proximal artery and main branch.
FIG. 3 is a cross section of the bifurcation showing a side branch guide wire being placed through the side of the main stent and through the ostium of the side branch.
FIG. 4 is a side view of a distal portion of a two guide wire stent delivery system for side branch stents showing the balloon in its inflated state.
FIG. 5A is a 3-dimensional view of the pre-deployed side branch stent as crimped onto the deflated balloon at the distal portion of the stent delivery system of FIG. 4.
FIG. 5B is a 3-dimensional view of the pre-deployed side branch stent as crimped onto the deflated balloon at the distal portion of the stent delivery system with a main guide wire tube that has a proximal portion attached to the outer sheath and a distal portion that is attached to the balloon.
FIG. 5C is a 3-dimensional view of the pre-deployed side branch stent as crimped onto the deflated balloon at the distal portion of the stent delivery system with a main guide wire tube that has a longitudinally extending central opening of the tube to enhance its flexibility.
FIG. 6 illustrates the side branch stent as it would be oriented and placed into the side branch of a stenosed bifurcation.
FIG. 7 is a cross section of the side branch stent after it is deployed into the side branch of the bifurcation.
FIG. 8 is a cross section of the distal portion of an alternative embodiment of a stent delivery system that has an inflated balloon into which a main guide wire tube has been placed.
FIG. 9 is a longitudinal cross section of an alternative embodiment of the invention using a highly compliant balloon onto which the side branch stent is mounted.
FIG. 10 is a transverse cross section of the balloon of the side branch stent delivery system prior to balloon inflation.
FIG. 11 is a transverse cross section of a conventionally folded balloon with an elastomer wrapping to prevent angular displacement of the side branch stent when the balloon is inflated.
FIG. 12 is a three-dimensional drawing of an alternative preferred embodiment of the present invention showing a portion of the main guide wire tube extending freely beyond the point where it is attached to the balloon.

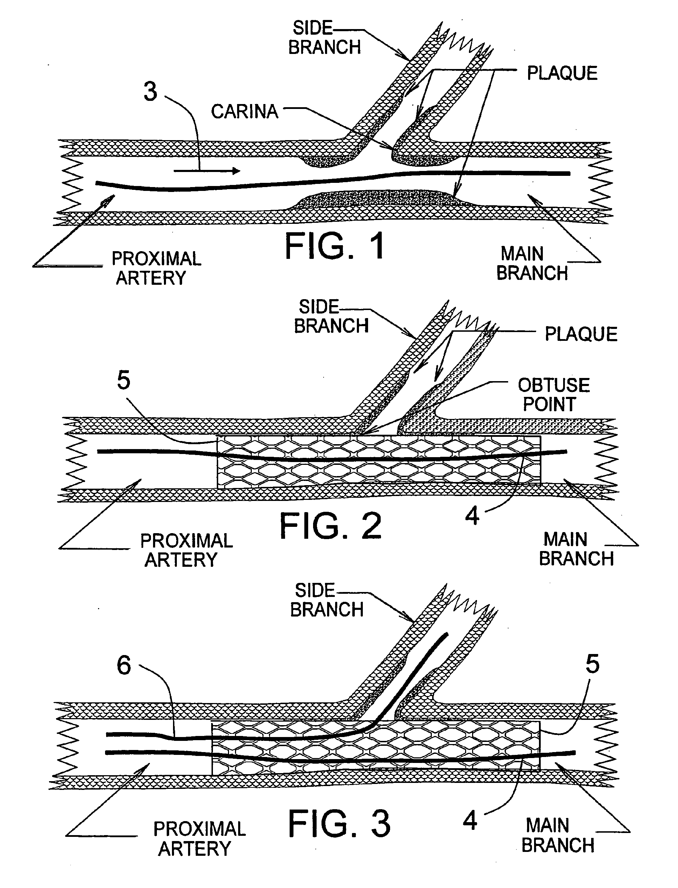

FIG. 1 is a cross section of a stenosed arterial bifurcation into which a main guide wire 4 has been placed. The arrow 3 shows the direction of blood flow. Figure 1 also shows the location of the proximal artery, and the main branch and side branch of the bifurcation. FIG. 1 also shows the typical location of plaque deposits within the bifurcation that prevent normal blood flow. Also shown in FIG. 1 is the carina or saddle point that is situated at the distal end of the ostium of the side branch.

FIG. 2 is a cross section of a main stent 5, as it would be placed by conventional means over the guide wire 4 and extending from a proximal artery into the main branch of that artery. FIG. 2 also shows the point that marks the proximal end of the ostium of side branch that can be termed the "obtuse point" because of the obtuse angle that the side branch makes with the proximal artery at that point.

FIG. 3 is a cross section of the bifurcation showing a side branch guide wire 6 placed through the side of the deployed main stent 5. After the guide wire 6 is placed, a dilation balloon (not shown) is advanced over the guide wire 6 and through the side of the main stent 5. The balloon is then inflated to form an approximately circular opening in the side of the main stent 5 to "unjail" " (or free the exposure of) the ostium of the side branch. The inflated unjailing balloon could also serve to pre-dilate the side branch artery prior to insertion of the side branch stent.

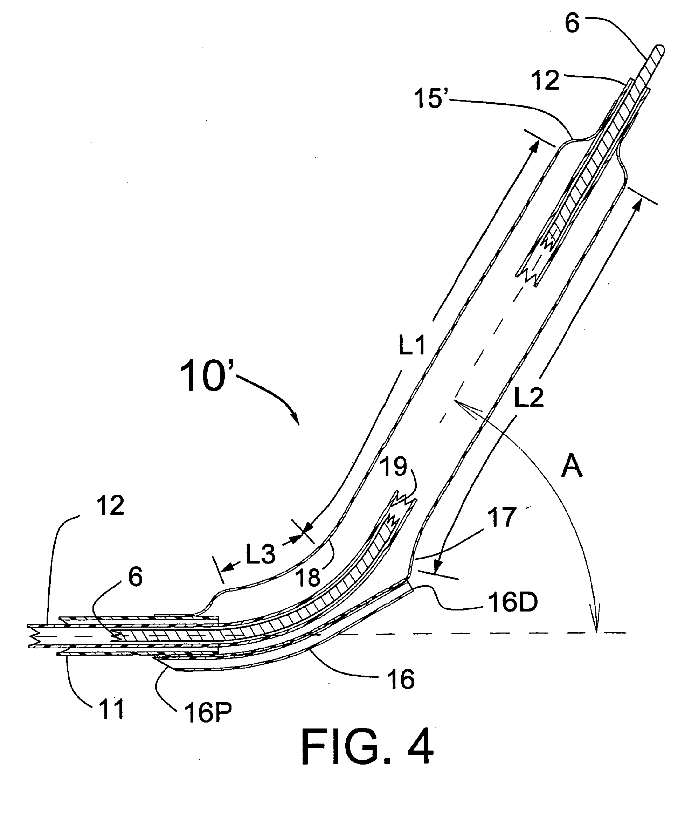

The design of the stent 5 is such that the cell size when it is deployed is optimally less than approximately 4.0 mm square and the cell perimeter length is optimally greater than approximately 10 mm. Even better is a cell size of less than 3.5 square mm and a cell perimeter of 11 mm, that when forced into a near circular shape by a dilatation balloon inflated when placed through the side of the stent 5 can create an opening diameter as large as 3.5 mm. The 3.5 mm diameter is sufficiently large to be larger than most of the ostium diameters where the opening in the side of the main stent 5 will be placed. This type of stent design is shown in FIGS. 1 and 4 of US-6540775. An important attribute of such a stent 5 is that a side opening can be made to a diameter of approximately 3.5 mm without fracturing any of the stent struts. This is highly desirable to avoid causing a broken strut to be thrust into the wall of the artery at the bifurcation.

FIG. 4 is a side view of the inflated balloon 15' of the stent delivery catheter 10' onto which the side branch stent will be crimped after the balloon 15' is deflated and folded. The inflated balloon 15' is shown as it is mounted onto a distal portion of the stent delivery system 10 for the side branch stent. The balloon 15' is joined at its distal end to an inner shaft 12 and joined at its proximal end to an outer shaft 11. The inner shaft 12 has a guide wire lumen 19. The balloon 15' has a uniform diameter for most of its length onto which the side branch stent will be mounted. The proximal end of the balloon 15' has a flared section 17 that will be placed at the carina of the side branch and a second flared section 18 that is to be placed at the obtuse point of the ostium of the side branch. Flaring near the proximal end of the balloon 15' extends around the entire circumference of the balloon 15' near its proximal end. Flaring allows the proximal end of the side branch stent to be pushed outward in the region of the ostium of the side branch for better apposition of the proximal portion of the stent by flaring outward that proximal portion against the walls of the arterial bifurcation.

The length of the side branch stent to be mounted on the balloon 15' would be approximately 3 mm longer on the side that goes to the obtuse point compared to the side that goes to the carina. A typical length L1 for the side branch stent would be approximately 18 ± 5 mm and the typical length L2 of the stent would be approximately 15 ± 5 mm. Ideally, the difference in length is adjusted so that when expanded by the balloon 15', the stent's proximal end will form the desired angle A with the longitudinal axis of the stent. The angle "A" is typically between 30 and 75 degrees, with 60 degrees being an optimal setting. The length L3 of the proximal section of the balloon 15' would be approximately 3 mm. Typical inflated diameters for the balloon 15' would be between 2 and 4 mm.

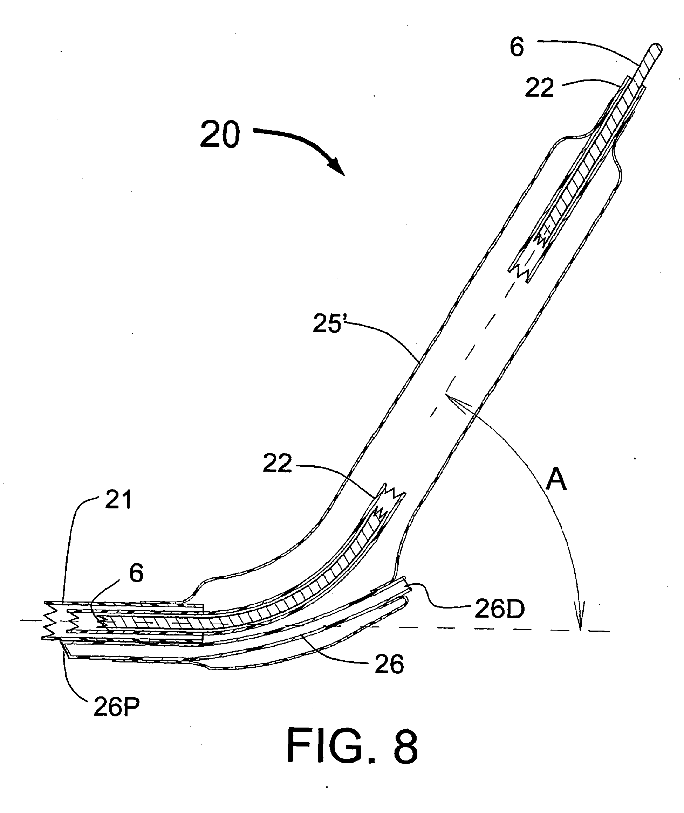

A unique feature of the balloon 15' is the fact that its proximal end is placed at an angle "A" relative to the distal portion of the balloon 15'. The advantage of this shape for the balloon 15' will be described later while examining FIG. 7. Another unique feature of the stent delivery system 10 is a flexible main guide wire tube 16 that has a proximal end 16P and a distal end 16D. Although FIG. 4 shows the tube 16 to have its proximal end located at a point on the outside of the outer shaft 11, a workable design would also have the tube 16 terminate with its proximal end attached to the balloon 15'. The distal end 16D of the tube 16, and preferably much of the length of the tube 16 is fixedly attached to the outside of the balloon 15'. The length of the tube 16 is preferably between approximately 0.5 and 5 cm although longer lengths are also envisioned.

FIG. 4 shows that there is a proximal opening of the main guide wire tube 16 at its proximal end 16P and a distal opening at the tube's distal end 16D. The guide wire tube 16 is designed to have the main guide wire 4 pass slideably through it as the side branch stent is advanced into the bifurcation. The purpose of this design is to urge the side branch stent to obtain the correct angular orientation as it is advanced into the side branch artery. The correct angular placement will cause the flared section 17 to become situated at the carina of the bifurcation and the flared section 18 to become situated at the obtuse point. Furthermore, the tube 16 cooperates with the main guide wire 4 to have the correct longitudinal placement of the side branch stent into the side branch.

Correct longitudinal placement occurs when the most proximal circumferential set of strut members of the deployed side branch stent is approximately coplanar with the ostium of the side branch. That is, the proximal circumferential set of strut members of the side branch stent neither extends into the lumen of the main stent 5, nor is it displaced several millimeters distally beyond the ostium of the side branch. Only when the proximal circumferential set of strut members of the side branch stent is placed approximately at the ostium of the side branch can the drug eluting from those strut members be effective in preventing arterial restenosis. That proper angular and longitudinal placement of the side branch stent is a most important goal of this invention. The placement of the distal end 16D of main guide wire tube 16 is to be in close proximity to the proximal end of the stent mounted on the balloon 15'. "Close proximity" means closer than approximately 2.5 mm and ideally less than 1.0 mm. This assures both the proper longitudinal position and angular orientation of the side branch stent within the side branch artery. This will be shown in additional detail with the assistance of FIG. 7.

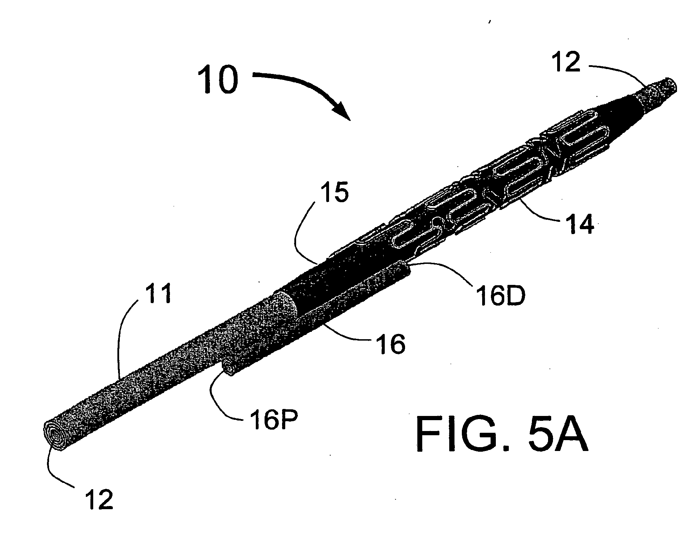

FIG. 5A is a 3-dimensional view of the distal portion of the stent delivery system 10 which includes the balloon 15 in its deflated state onto which is mounted an angulated side branch stent 14. Also shown in FIG. 5A is the outer shaft 11 and inner shaft 12 onto which the balloon 15 is mounted. FIG. 5A also shows the main guide wire tube 16 having a proximal end 16P and a distal end 16D. The guide wire tube 16 is designed to have the main guide wire 4 pass through it as the side branch stent 14 is advanced into the side branch of the bifurcation.

It should be noted that the stent delivery system 10 in its pre-deployed state as shown in FIG. 5A, could have the outer shaft 11 essentially aligned with the balloon 15. This can be accomplished because of the small diameter of the balloon 15 prior to inflation and the absence of any pressure in the balloon 15. A near straight geometry for the distal portion of the stent delivery system 10 is desirable when advancing it into the body's vascular system. Some tendency to bend in the proximal portion of the balloon 15 prior to stent deployment would still be satisfactory for delivering the stent 14 into the side branch artery.

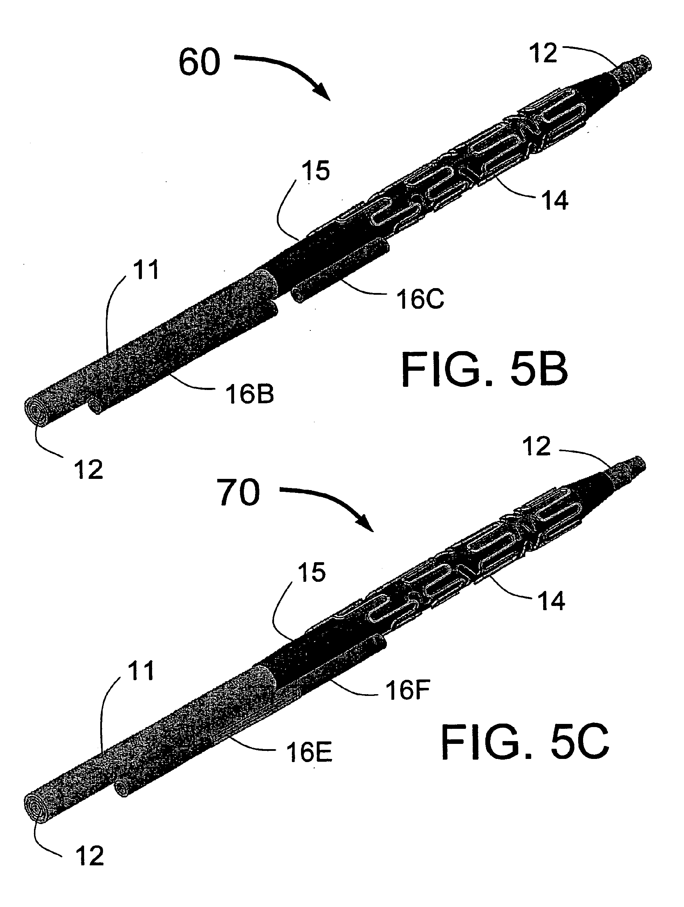

FIGS. 5B and 5C illustrate alternate means for placing a main guide wire tube onto the stent delivery system. Specifically, FIG. 5B shows a main guide wire tube proximal portion 16B attached to the outer shaft 11 and a distal portion 16C that is attached to the balloon 15. FIG. 5C shows a continuous main guide wire tube 16F that has a longitudinally elongated hole 16E located between the tube's proximal and distal portions. The purpose of the hole 16E is to increase the flexibility of the tube 16F in that region. The hole 16E could have an arc length between 90 and 330 degrees. The greater the arc length of the opening 16E, the greater would be the flexibility of the guide wire tube 16F.

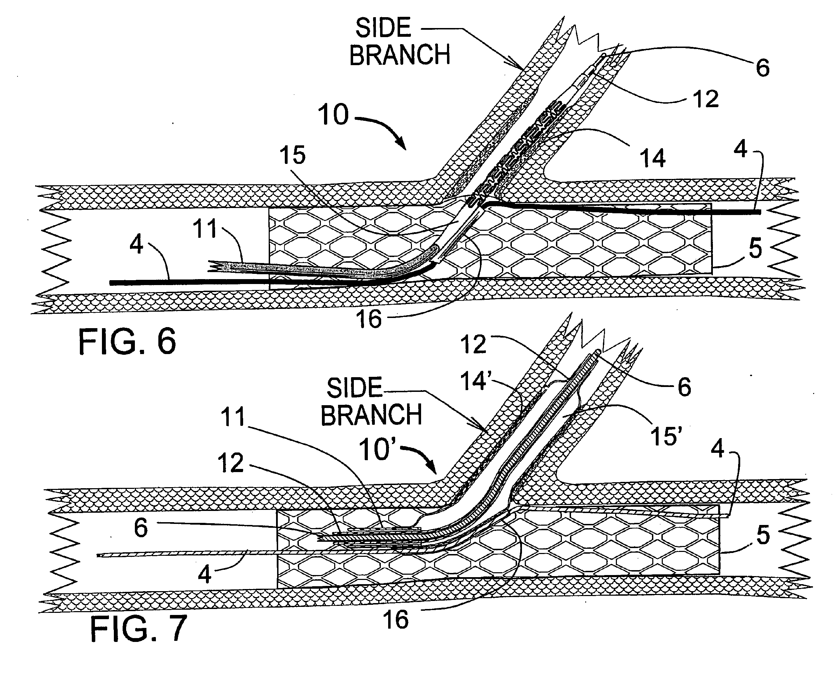

FIG. 6 illustrates the side branch stent delivery system 10 causing the pre-deployed side branch stent 14 to be properly positioned at the ostium of a side branch vessel. As shown in FIG. 3, both the main guide wire 4 and the side branch guide wire 6 are placed respectively into the main branch and the side branch of the bifurcation prior to advancing the stent delivery system 10 through the patient's vascular system. The stent delivery system 10 has the inner shaft 12 being advanced over the side branch guide wire 6 while the main guide wire tube 16 is simultaneously advanced over the main guide wire 4. Because the diameter of the pre-deployed side branch stent 14 is quite small (typically about one mm) and because the main stent 5 has already been expanded radially outward against the wall of the proximal artery, the distal portion of the stent delivery system 10 is quite free to rotate within the lumen of the deployed main stent 5. This causes the main guide wire 4 where it exits at the distal end 16D of the tube 16 to be forced into close proximity to the carina of the bifurcation as the stent delivery system 10 is advanced. The forcing of the distal end 16D of the tube 16 and the main guide wire where it exits the tube 16 against the carina has three beneficial effects. First, the proper angular orientation of the side branch stent 14 is assured prior to its deployment against the walls of the side branch. Second, the correct longitudinal position of the stent 14 is assured. That is, the most proximal circumferential sets of strut members of the side branch stent 14 will be placed approximately coplanar with the ostium of the side branch, and third, pushing the stent delivery system 10 against the carina will prevent (or reduce) the rotation of the most proximal circumferential set of strut members of the stent 14 as the balloon is inflated so as to maintain the desired angular alignment of the stent's proximal end with the plane of the ostium.

To assist the interventionalist in placing the stent delivery system 10 in an optimum angular orientation prior to causing the distal end 16D of the tube 16 to be situated at the carina, it will be advisable to have the tube 16 placed in close proximity to the side of the main stent 5 that is opposite the ostium of the side branch. This orientation of the tube 16 is shown in both FIGS. 6 and 7. By making the tube 16 radiopaque, the user will more easily be able to cause the orientation of the tube 16 to be as shown in FIGS. 6 and 7. That is, after the distal end of the stent delivery system 10 is first placed through the side opening in the main stent 5, the user rotates the stent delivery system 10 so that the tube 16 is situated on the side of the outer shaft 11 and balloon 15 that is away from the ostium of the side branch. By this means, less will be required of the cooperation between the main guide wire 4 and the stent delivery system 10 to achieve the proper angular orientation of the stent 14 as is shown in FIG. 6.

FIG. 7 is a cross section of the bifurcation which shows the inflated balloon 15' causing the deployed side branch stent 14' to be placed in close apposition to the walls of the side branch artery. Also seen in FIG. 7 is the fact that the outer shaft 11 will no longer have its longitudinal axis lying parallel to the longitudinal axis of the balloon 15' as is shown in FIG. 5A when there is no pressure in the balloon 15. When the balloon 15' is inflated to a high pressure to deliver the stent 14' against the walls of the side branch artery, the balloon 15' will be forced into substantially the shape in which it was formed as shown in FIG. 7. This shape is ideal for placing the stent 14' into the side branch artery and is one of the several novel features of this invention. It should be understood that part of the proximal portion of the inflated balloon 15' could be in contact with the side of the main stent 5 that is opposite from the ostium of the side branch artery. Also, the guide wire tube 16 could also be placed in contact with that side of the main stent when the balloon IS' is inflated.

Although the proximal end of the stent 14' as shown in FIG. 7 is situated near the obtuse point of the bifurcation without entering the main stent 5, it should be understood that that proximal portion of the stent 14' could be extended further into the main stent 5 to assure optimum coverage of the ostial region of the side branch artery. Specifically, the proximal end of the stent 14' could extend into the stent 5 so that there would be regions on the wall of the bifurcated artery where there are two layers of the metal, one layer from the stent 5 and the second layer from the stent 14'. This could be accomplished for all regions of the flared proximal end of the stent 14' from the obtuse point to the carina.

FIG. 8 is an alternate embodiment of this invention showing a stent delivery system 20 having an outer shaft 21, an inner shaft 22, an inflated balloon 25' and a main guide wire tube 26 having a proximal end 26P and a distal end 26D. Instead of being fixedly attached to the outside of the balloon 15' as shown in FIG. 4, the tube 26 of FIG. 8 is placed within the balloon 25'. For this configuration, the tube 26 must be sealed where it enters and exits the balloon 25' so that the balloon 25' can be inflated to a high pressure.

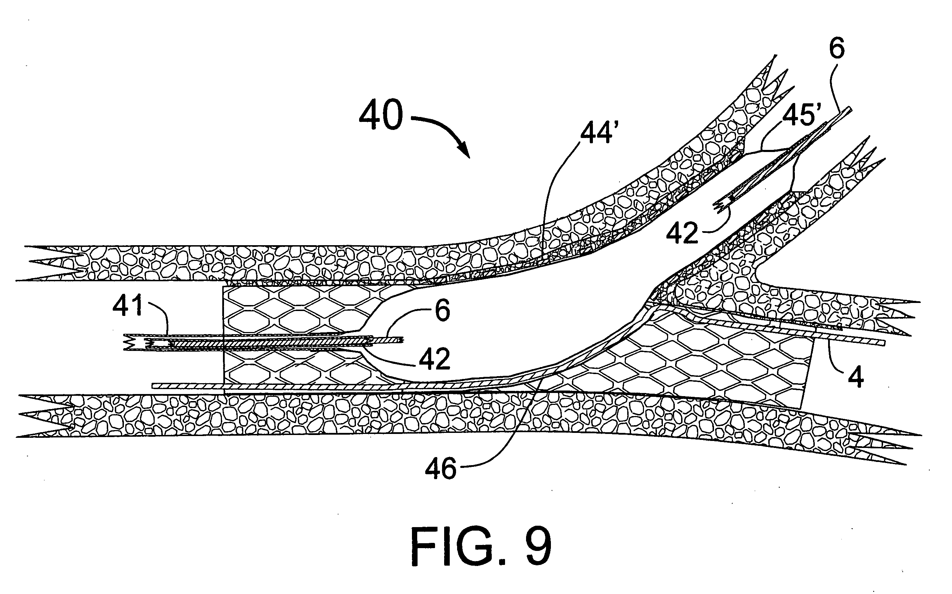

FIG. 9 is another alternate embodiment of the invention showing a stent delivery system 40 having an outer shaft 41, inner shaft 42 that goes over the side branch guide wire 6 and a guide wire tube 46 that can be advanced over the main guide wire 4. FIG. 9 also shows an inflated balloon 45' onto which an angulated side branch stent 44' has been deployed against the walls of the side branch including its ostial region. One main difference between the balloon 45' as compared to the balloon 15' of FIGS. 4 and 7 is that the balloon 45' would be a highly compliant balloon which is essentially symmetric around its longitudinal axis. The balloon 15' might (for example) have a change in diameter of only 0.10 mm from an inflation pressure of 10 atm. to an inflation pressure of 12 atm. The highly compliant balloon 45' might have a diameter change of approximately 0.25 mm to as much as 1.0 mm as the balloon inflation pressure is increased from 10 atm. to 12 atm. Therefore, as seen in FIG. 9, that part of the balloon 45' that is not confined by the walls of the side branch artery could readily enlarge to a larger diameter within the main proximal artery. This type of highly compliant balloon 45' would be ideal for causing the proximal portion of the stent 44' to cover the ostial region of the side branch artery. To make certain of good coverage of the obtuse point region of the ostium, the stent 45' could have an extended length along that side of the stent as opposed to a lesser length of the side of the stent 44' that goes to the carina region of the ostium of the side branch.

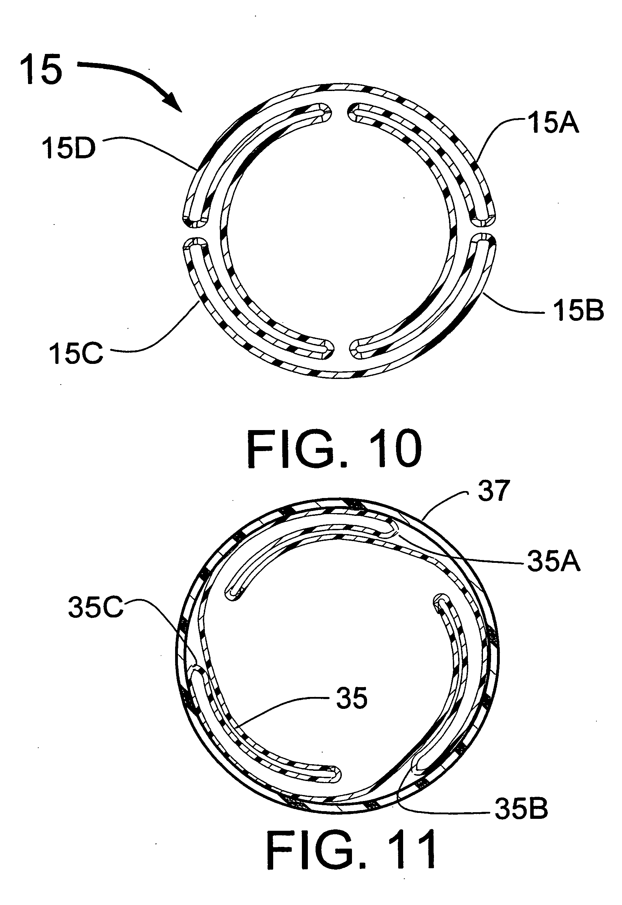

FIG. 10 is a transverse cross section of the balloon 15 (similar to that of the cross section of balloon 25) in its deflated state, which is the state it is in when a stent is crimped onto the balloon. This cross section does not show the inner shaft or a guide wire in that inner shaft. It should be noted that the balloon folds 15A and 15B are symmetrical and the folds 15C and 15D are also symmetrical. This folding configuration decreases any propensity of a stent mounted on the balloon to rotate about the balloon's longitudinal axis when the balloon is inflated. Such a balloon will further enhance the ability of the stent delivery system 10 of FIG. 6 to maintain the angular alignment of the most proximal circumferential set of strut members of the stent 14 as the balloon 15 expands it into the side branch vessel.

FIG. 11 is a transverse cross section of a deflated balloon 35 in which all the balloon folds, 35A, 35B and 35C, are folded in the same direction. A stent mounted on a balloon that is folded in this way would have some angular rotation about the longitudinal axis of the balloon when it is expanded. Rotation upon balloon inflation would tend to reduce the ability of the present invention to have the most proximal circumferential sets of strut members of the deployed stent be approximately co-planar with the ostium of the side branch artery. However, if an elastomer tube 37 is wrapped around the balloon 37, and the stent is crimped onto that tube 37, then there will be a decreased tendency for the stent to rotate about the longitudinal axis of the balloon 35 when the balloon is inflated. This system would work particularly well if the exterior surface of the balloon 35 and the interior surface of the tube 37 were each coated with an agent to improve lubricity. The elastomer tube 37 could be made from a highly elastic material such as silicone or natural rubber.

It should also be understood that for both the balloons 15 and 35 that the edges of each fold of the balloons would form a line that is parallel to the balloon's longitudinal axis. That is, the edges of the folds would not form a helical pattern, as is frequently the case for stent deployment balloons and PTCA balloons.

Although the most frequent use of the invention as described herein would be for the treatment of stenosed bifurcated arteries, it should be understood that the invention could also be applied to other bifurcated vessels of the human body.

Although the main stent and the side branch stent as described herein could be free of any drug coating, it should be understood that an optimum design would include a coating from which one or more drugs would elute. Some of the purposes of such drugs would be to reduce arterial restenosis and to prevent sub-acute thrombosis. To that end, the drug(s) for placement onto the stent could be selected from the group consisting of cytostatic drugs, cytotoxic drugs, anti-thrombogenic drugs, sirolimus, anti-sense to c-myc (Resten-NG), tacrolimus (FK506), everolimus and other analog of sirolimus and everolimus including: SDZ-RAD, CCI-779, 7-epi-rapamycin, 7-thiomethyl-rapamycin, 7-epi-trimethoxyphenyl-rapamycin, 7-epi-thiomethyl-rapamycin, 7-demethoxy- rapamycin, 32-demethoxy, 2-desmethyl, prolene, heparin and paclitaxel. A particularly valuable drug combination for coating the main stent and side branch stent would be a cytostatic drug and an anti-thrombogenic drug. An example of such a combined drug coating would be sirolimus and heparin.

One method for stenting an arterial bifurcation in a human patient using the invention described herein may be as follows:
placing a main guide wire into and through a proximal artery and a main branch of that artery; advancing a stent delivery system that includes a main branch stent over the guide wire and deploy the main stent with its longitudinal center located approximately over the ostium of a bifurcation of the proximal artery;
inserting a side branch guide wire through the side of the deployed main stent;
advancing a balloon angioplasty catheter over the side branch guide wire until the balloon located at a distal portion of the balloon angioplasty catheter is centered within the ostium of the side branch artery and inflate the balloon to open the struts of the main stent to form an opening at the ostium of the side branch artery that is approximately the same area as the ostium of the side branch artery;
deflating the balloon from step d) and removing the balloon angioplasty catheter from the human subject;
advancing a side branch stent delivery system having two guide wire lumens over both the side branch guide wire and the main guide wire, the stent delivery system having an inflatable balloon and an angulated side branch stent both located at a distal portion of the stent delivery system, the stent delivery system also having a main guide wire tube fixedly attached to the balloon at a location that is just proximal to the proximal end of the side branch stent, the side branch guide wire passing through the inner shaft of the stent delivery system and the main guide wire passing through the main guide wire tube that is fixedly attached to the balloon; advancing the stent delivery system for the side branch stent until the stent's proximal end is placed at the carina of the bifurcation;
inflating the balloon so as to deploy the side branch stent against the walls of the side branch of the bifurcation;
deflating the balloon and removing the side branch stent delivery system from the human subject; and
removing the side branch guide wire and the main guide wire from the human subject.

The method described above can also include pre-dilatation of the proximal artery and main branch artery prior to placement of the main stent. Another added step could be the post-dilatation of both the main stent and the side branch stent to obtain improved apposition of the stents to the walls of the arteries into which they are placed. This post-dilatation can be accomplished with separate balloons, two "kissing" balloons or a single "Y"-shaped balloon.

A second method for stenting an arterial bifurcation in a human patient using the invention described herein may be as follows:
inserting a side branch guide wire through the arterial side branch;
advancing a main guide wire through the main proximal artery and into the main branch;
advancing a side branch stent delivery system having two guide wire lumens over both the side branch guide wire and the main guide wire, the stent delivery system having an inflatable balloon and an angulated side branch stent both located at a distal portion of the stent delivery system, the stent delivery system also having a main guide wire tube with an attached portion fixedly attached to the balloon with the distal end of the attached portion being located just proximal to the proximal end of the side branch stent and the main guide wire tube having a second portion that extends unattached to the balloon and extends freely in a distal direction beyond the distal end of the attached portion, the side branch guide wire passing through the inner shaft of the stent delivery system and the main guide wire passing through the main guide wire tube;
advancing the stent delivery system for the side branch stent until the stent's proximal end is situated at the carina of the bifurcation;
inflating the balloon so as to deploy the side branch stent against the walls of the side branch artery of the bifurcation;
deflating the balloon and removing the side branch stent delivery system and the side branch guide wire from the human subject;
advancing a stent delivery system that includes a main stent over the main guide wire and deploying the main stent with its longitudinal center located approximately over the ostium of the bifurcation of the proximal artery;
advancing a side branch guide wire through the main stent at the ostium of the arterial side branch and advancing the side branch guide wire until it extends for at least one centimeter into the arterial side branch;
advancing a balloon angioplasty catheter over the side branch guide wire until the balloon located at a distal portion of the balloon angioplasty catheter is approximately centered within the ostium of the side branch artery and inflating the balloon to open the struts of the main stent to form an opening at the ostium of the side branch artery that is approximately the same area as the area of the ostium of the side branch artery;
deflating the balloon from step i) above and removing the balloon angioplasty catheter from the human subject; and
removing the side branch guide wire and the main guide wire from the human subject.
The method described immediately above can also include pre-dilatation of the side branch artery prior to placement of the side branch stent and pre-dilatation of the proximal artery and main branch artery prior to placement of the main stent. Another added step could be the post-dilatation of both the main stent and the side branch stent to obtain improved apposition of the stents to the walls of the arteries into which they are placed. This post-dilatation can be accomplished with a single balloon used once or twice, two "kissing" balloons or a single "Y"-shaped balloon.

In the stent delivery system embodiments shown herein, the side branch guide wire lumen is coaxial within the balloon and has a distal opening at the distal end of the stent delivery system. It is envisioned that the proximal end of the side branch guide wire lumen (i.e. the lumen contained within the inner shaft 12 of FIG. 4) could be at the proximal end of the stent delivery system making the stent delivery system, an "over-the-wire" device. Or, the proximal end of the side branch guide wire lumen might be at some point between the proximal end of the balloon and the proximal end of the stent delivery system, making the stent delivery system a "monorail" or rapid exchange type device.

It should also be understood that in place of the side branch guide wire lumen within the inner shaft 12 of FIG. 4, a fixed guide wire as described by Fischell et al in US-6375660 could be used. Alternatively, a guide wire lumen through an elongated distal tip of the stent delivery system could provide passage for the side branch guide wire. Fischell et al in US-5830227 describe this design concept for a catheter tip allowing passage of a guide wire.

Although the stent shown mounted on the stent delivery system of FIGS. 5 through 7 has an angulated proximal end, it is envisioned that a standard stent with a proximal circumferential set of strut members perpendicular to the longitudinal axis of the stent would also work and may be preferable for side branches at angles near to 90 degrees. When used for a side branch such a conventional stent can also be called a "side branch stent".

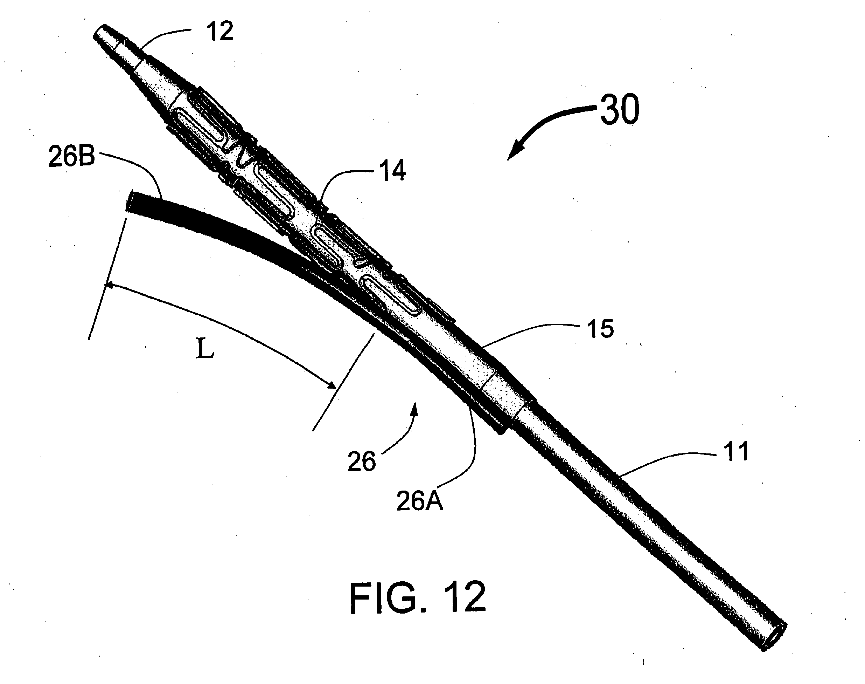

FIG. 12 is a three-dimensional illustration of another preferred embodiment of the present invention. FIG. 12 shows a side branch stent delivery system 30 that includes an outer shaft 11, inner shaft 12, stent 14 and an inflatable balloon 15. Each of these elements of the stent delivery system 30 is essentially identical to those same elements that are shown in FIG. 5. In place of the main guide wire tube 16 of the stent delivery system 10 of FIG. 5, the stent delivery system 30 has a main guide wire tube 36 that has a proximal portion 36A and a distal portion 36B. The proximal portion 36A is fixedly attached to the balloon 15 and electively also attached to the outer shaft 11. The distal end of the proximal portion 36A is attached to the balloon 15 just proximal to the proximal end of the stent 14 at a position where that proximal end of the stent 14 will be placed at the carina of the bifurcation. The distal attachment point of the proximal portion 36A should be within approximately 3 mm from the proximal end of the stent 14 and ideally within 1 mm of the proximal end of the stent 14. The distal portion 36B that is not attached to any part of the stent delivery system 30 forms a continuous tube with the attached, proximal portion 36A. The advantage of the design of FIG. 12 over the design of FIG. 5 is that the unattached, distal portion 36B of the main guide wire tube 36 provides added support for the main guide wire in assuring the accurate longitudinal position and angular orientation of the side branch stent 14 within the arterial side branch. A suitable length "L" for the distal, unattached portion 36B would be between approximately 2 and 30 mm. An ideal length "L" would be between approximately 10 and 15 mm. Any method for stenting a stenosis at a bifurcation could use any of the stent delivery systems 10, 20 30, 60 or 70 as described herein.

Various other modifications, adaptations and alternative designs are of course possible in light of the teachings as presented herein. Therefore it should be understood that, while still remaining within the meaning of the appended claims, this invention could be practiced in a manner other than that which is specifically described herein.

## Claims

1. A system for placing a stent into the side branch of an arterial bifurcation, the system including:
an inflatable balloon located at a distal portion of a stent delivery system, the stent delivery system having an outer shaft with a longitudinal length, the outer shaft having a distal end that is fixedly attached to the proximal end of the balloon;
a pre-deployed side branch stent mounted coaxially onto the balloon for placement into the side branch of an arterial bifurcation, the stent having a proximal end and a distal end;
a guide wire; and
a main guide wire tube having a proximal end and a distal end and having an interior lumen that allows the stent delivery system to be moved slideably over the guide wire, the main guide wire tube being fixedly attached to the balloon at a location that is in close proximity to the proximal end of the stent.

2. A system for placing a stent into the side branch of an arterial bifurcation, the system including:
an inflatable balloon located at a distal portion of a stent delivery system, the stent delivery system having an outer shaft with a longitudinal length, the outer shaft having a distal end that is fixedly attached to the proximal end of the balloon;
a pre-deployed stent mounted coaxially onto the balloon for placement into the side branch of an arterial bifurcation, the stent having a proximal end and a distal end;
a guide wire; and
a main guidewire tube that allows the stent delivery system to be moved slideably over the guide wire, the main guide wire tube having a proximal portion having a distal end and the main guide wire tube also having a distal portion which is not directly connected to the stent delivery system, the distal portion extending continuously in a distal direction from the distal end of the proximal portion, the distal end of the proximal portion being fixedly attached to the balloon at a point that is in close proximity to the proximal end of the stent.

3. The system of claim 1 where the guidewire tube extends in a generally longitudinal direction onto a proximal portion of the balloon.

4. The system of claim 1 where the guidewire tube extends in a proximal direction onto the outer shaft of the stent delivery system.

5. The system of claim 1 where at least part of the guide wire tube is fixedly attached to the outside of the balloon.

6. The system of claim 1 or 2 where at least part of the guidewire tube is located within the balloon.

7. The system of claim 1 or 2 where the guide wire tube is made from a flexible elastomer that is fixedly attached along its entire length to both the balloon and the outer shaft.

8. The system of claim 1 where the guide wire tube has a distal opening at its distal end, the distal opening being situated within a distance of less than 1.0 mm from the proximal end of the stent.

9. The system of claim 1 where the guide wire tube is formed in two separate portions, a first portion being a proximal portion that is fixedly attached to the outer shaft of the stent delivery system and a second portion that is a distal portion that is fixedly attached to the balloon.

10. The system of claim 1 where the guide wire tube is formed with a longitudinal opening situated between a first portion of the tube that is a proximal portion that is fixedly attached to the outer shaft of the stent delivery system and a second portion of the tube that is a distal portion that is fixedly attached to the balloon.

11. The system of claim 1 where the stent has a multiplicity of circumferential sets of strut members, the normal to the plane of the most proximal circumferential set of strut members making an angle that is greater than 20 degrees relative to the longitudinal axis of the balloon.

12. The system of claim 1 or 2 where the stent is coated with at least one drug whose effect is to decrease restenosis at the site of the arterial bifurcation by elution of the at least one drug into the wall of the artery in the region where the stent is placed.

13. The system of claim 12 where the drug is selected from the group consisting of a cytostatic drug, a cytotoxic drug, sirolimus, anti-sense to c-myc (Resten-NG), tacrolimus (FK506), everolimus and other analogs of sirolimus or everolimus including: SDZ-RAD, CCI-779, 7-epi-rapamycin, 7-thiomethyl-rapamycin, 7-epi-trimethoxyphenyl-rapamycin, 7-epi-thiomethyl-rapamycin, 7-demethoxy- rapamycin, 32-demethoxy, 2-desmethyl, proline and paclitaxel.

14. The system of claim 12 or 13 where there is an increased amount of the at least one drug on a proximal portion of the stent as compared to the amount of drug on a distal portion of the stent.

15. The system of claim 1 or 2 where the stent is coated with a drug for the purpose of decreasing subacute thrombosis.

16. The system of claim 15 where the drug is heparin.

17. The system of claim 1 where the balloon when inflated has a generally uniform diameter for most of its length and an outward flare to a larger diameter where the inflated balloon is situated under the proximal end of the stent.

18. The system of claim 1 where the balloon when inflated has a generally uniform diameter for most of its length and is a compliant balloon that readily expands to a greater diameter when not confined by the walls of the side branch artery.

19. The system of claim 1 where the length of the stent is shorter on the side where the deployed stent's proximal end is situated at the carina of the bifurcation and longer on the side of the deployed stent that reaches the obtuse point of the bifurcation, the difference in length being greater than 1.0 mm.

20. The system of claim 1 further including a main branch stent, the main branch stent having the area for each cell of the deployed stent that is less than 4.0 mm² and each cell having a perimeter length that is greater than 10 mm.

21. The system of claim 1 where the stent delivery system also includes an inner shaft having a guide wire lumen where the distal end of the guide wire lumen is located at the distal end of the stent delivery system.

22. The system of claim 1 where the stent delivery system also includes an elongated distal tip placed distal to the balloon, the distal tip having a guide wire lumen, the distal end of the guide wire lumen being located at the distal end of the distal tip and the proximal end of the guide wire lumen being located between the distal end of the balloon and the distal end of the distal tip.

23. The system of claim 1 where the distal end of the stent delivery system includes a fixed guide wire attached to the distal portion of the stent delivery system, the fixed guide wire extending in a direction distal to the balloon.

24. The system of claim 2 where the distal end of the proximal portion of the main guide wire tube is attached to the balloon within a distance of less than 3.0 mm from the proximal end of the stent.

25. A pre-deployed balloon mounted at a distal portion of a catheter shaft the pre-deployed balloon being folded with an even number of folds formed as pairs, each pair of folds being symmetrically located around the circumference of the balloon.

26. The balloon of claim 25 where there are exactly two folds.

27. The balloon of claim 25 where there are exactly four folds.

28. A means to prevent the rotation of a stent mounted on an inflatable balloon, the means including placing an elastomer tube around the balloon, the elastomer tube expanding elastically as the balloon is inflated.

29. The means of claim 28 where both the interior surface of the elastomer tube and the external surface of the balloon are each treated with a chemical to increase their lubricity.
